(19) Europäisches Patentamt / European Patent Office / Office européen des brevets

(11) **EP 1 815 820 A1**

(12) **EUROPEAN PATENT APPLICATION**
published in accordance with Art. 158(3) EPC

(43) Date of publication:
08.08.2007 Bulletin 2007/32

(51) Int Cl.:
*A61F 2/06* (2006.01)    *A61L 27/00* (2006.01)

(21) Application number: 05811444.8

(22) Date of filing: 18.11.2005

(86) International application number:
PCT/JP2005/021638

(87) International publication number:
WO 2006/054799 (26.05.2006 Gazette 2006/21)

(84) Designated Contracting States:
AT BE BG CH CY CZ DE DK EE ES FI FR GB GR
HU IE IS IT LI LT LU LV MC NL PL PT RO SE SI
SK TR

(30) Priority: 19.11.2004 JP 2004335980
10.06.2005 JP 2005170858

(71) Applicant: TEIJIN LIMITED
Osaka-shi, Osaka 541-0054 (JP)

(72) Inventors:
• KITAZONO, Eiichi
Tokyo Research Center Teijin Ltd.
Hino-shi, Tokyo 191-0065 (JP)

• KANEKO, Hiroaki,
Tokyo Research Center Teijin Ltd.
Hino-shi, Tokyo 191-0065 (JP)
• MIYOSHI, T.,
Iwakuni Research Center TeijinLtd.
Iwakuni-shi, Yamaguchi 740-0014 (JP)
• KOMURA, Shinya
Iwakuni Research Center Teijin Ltd.
Iwakuni-shi, Yamaguchi 740-0014 (JP)
• SAITO, Ryo
Kita-ku, Tokyo 114-0003 (JP)

(74) Representative: Hallybone, Huw George et al
Carpmaels and Ransford
43-45 Bloomsbury Square
London WC1A 2RA (GB)

(54) **CYLINDRICAL MEMBER AND PROCESS FOR PRODUCING THE SAME**

(57) It is an object of the present invention to provide a cylindrical body which has an excellent elastic modulus and elastic recovery and is suitable for use as a scaffold material for revascularization. It is a further object of the present invention to provide a cylindrical body which has an outermost layer with high air permeability, is excellent in cell infiltration and is suitable for use as a scaffold material for revascularization.

The cylindrical body of the present invention is a hollow cylindrical body consisting of a plurality of concentric layers and having an outer diameter of 0.5 to 50 mm and a thickness of 200 to 5, 000 $\mu$m, and each layer is made of aliphatic polyester fibers having an average fiber diameter of 0.05 to 50 $\mu$m.

Fig. 6

EP 1 815 820 A1

**Description**

TECHNICAL FIELD

The present invention relates to a cylindrical body having a plurality of layers made of aliphatic polyester fibers and a manufacturing method thereof.

BACKGROUND ART

As an approach to the treatment of a greatly damaged or lost body tissue, researches into regeneration medicine for re-constructing an original body tissue making use of the differentiation and multiplication abilities of cells are being carried out energetically. The regeneration of a blood vessel is one of the researches, and researches into the regeneration of a blood vessel by cutting off a blood vessel which was damaged congenitally or by a disease and crosslinking a scaffold material for regeneration in a damaged part are under way.

As this scaffold material, there is proposed a vessel prosthetic material comprising polyurethane fibers and having openings with an inner diameter of 0.3 to 3 cm (patent document 1). However, a biodegradable material is preferred for regeneration medicine.

Study on a scaffold material comprising nanofibers obtained by an electrospinning process is under way (non-patent document 1). The nanofiber has a similar length and diameter as an extracellular matrix. Since the nanofiber has a small fiber diameter, it has a large specific surface area which is 100 times or more that of an ordinary fiber. Thereby, it has an advantage that cell adhesion is excellent. Therefore, a nanofiber structure is expected as an excellent scaffold material in regenerative medicine.

However, scaffold materials comprising nanofibers which have been studied up till now are uniform porous materials and greatly differ from a blood vessel which is a body tissue in structure. That is, the actual blood vessel does not have a uniform structure and consists of an inner layer including endothelial cells covering the inner wall of the blood vessel, an intermediate layer composed of smooth muscle cells and an outer layer composed of fibroblasts. The inner layer has a dense structure with a small space volume to control the selective permeation of a substance, the intermediate layer has an elastic function special to the blood vessel, and further the outer layer has a sparse structure with a large space volume to take a nutritive substance from the outside of the blood vessel.

There is proposed an artificial blood vessel including nonwoven cloth made of polymer fibers for medical use such as collagen or polyurethane having an outer diameter of several nm to several tens of $\mu$m (patent document 2).
(Patent Document 1) JP-A 52-110977
(Patent Document 2) JP-A 2004-321484
(Non-patent Document 1) Biomaterials, 25, 877 (2004)

DISCLOSURE OF THE INVENTION

It is an object of the present invention to provide a cylindrical body which has an excellent elastic modulus and elastic recovery and is suitable as a scaffold material for the regeneration of a blood vessel. It is another object of the present invention to provide a cylindrical body which has an outermost layer with high air permeability, is excellent in cell infiltration and is suitable for use as a scaffold material for the regeneration of a blood vessel.

The inventors of the present invention have studied a material which has a similar structure to a blood vessel, has the same level of mechanical strength as that of the blood vessel and is suitable for use as a scaffold material for the regeneration of the blood vessel. As a result, they have found that a cylindrical body consisting of a plurality of layers, manufactured by winding fibers formed from spinning of dopes containing an aliphatic polyester by the electrospinning process, has the same level of mechanical strength as that of a blood vessel and is suitable for use as a scaffold material for the regeneration of the blood vessel. The present invention has been accomplished based on this finding. They have also found that when a static eraser is used to form the outermost layer in the electrospinning process, a layer having a low fiber density, high air permeability and a space volume as large as the outer layer of the blood vessel of a living body is obtained. Thus, the present invention has been accomplished based on this finding.

That is, the present invention is a hollow cylindrical body consisting of a plurality of concentric layers and having an outer diameter of 0.5 to 50 mm and a thickness of 200 to 5,000 $\mu$m, wherein each of the layers is made of aliphatic polyester fibers having an average fiber diameter of 0.05 to 50 $\mu$m.

Further, the present invention is a method of manufacturing a hollow cylindrical body consisting of a plurality of concentric layers, comprising the steps of:

(i) preparing dopes, each containing an aliphatic polyester and a volatile solvent, corresponding to the number of layers;

(ii) forming fibers from the dopes by an electrospinning process and winding them up onto a collector to obtain single-layer cylindrical bodies corresponding to the number of layers; and
(iii) laminating together the obtained cylindrical bodies.

Further, the present invention is a method of manufacturing a hollow cylindrical body consisting of a plurality of concentric layers, comprising the steps of:

(i) preparing dopes, each containing an aliphatic polyester and a volatile solvent, corresponding to the number of layers;
(ii) forming layers from a first dope by an electrospinning process and winding them up onto a collector to form a layer; and
(iii) forming a layer from the next dope on the obtained layer.

BRIEF DESCRIPTION OF THE DRAWING

Fig. 1 shows an example of the apparatus used in an electrospinning process;
Fig. 2 shows another example of the apparatus used in the electrospinning process;
Fig. 3 is a perspective view of the apparatus shown in Fig. 2;
Fig. 4 shows still another example of the apparatus used in the electrospinning process;
Fig. 5 shows a further example of the apparatus used in the electrospinning process;
Fig. 6 is a diagram showing the section of the cylindrical body of the present invention;
Fig. 7 is a diagram showing the section of a bellows-like cylindrical body;
Fig. 8 shows a photo of the appearance of the cylindrical body obtained in Example 4; and
Fig. 9 shows a photo of the section of the cylindrical body obtained in Example 4.

Explanations of letters or notations

1. nozzle
2. dope
3. storage tank
4. ejection-side electrode
5. collection-side electrode
6. high-voltage generator
7. collector
8. static eraser
9. mountain portion
10. valley portion
11. outer diameter
12. interval between adjacent mountain portion
13. depth of the valley portion
14. thickness
15. inner diameter

Effect of the Invention

The cylindrical body of the present invention consists of a plurality of layers and has a similar structure to the blood vessel. The cylindrical body of the present invention has an excellent elastic modulus and elastic recovery, i.e., the same levels of elastic modulus and elastic recovery as those of a vascular tissue. Therefore, the cylindrical body of the present invention is suitable for use as a culture medium for vascular tissues. A cylindrical body having the innermost layer and/or the outermost layer made of aliphatic polyester fibers composed of a polymer containing a recurring unit derived from glycolic acid according to an embodiment of the present invention is excellent in cell adhesion and is suitable for use as a culture medium for vascular tissues. A cylindrical body having the outermost layer with high air permeability according to another embodiment of the present invention is similar in structure to the blood vessel of a living body having an outer film with a large space volume. Therefore, the cylindrical body is excellent in cell infiltration and suitable for use in the regeneration of vascular tissues.

BEST MODE FOR CARRYING OUT THE INVENTION

The present invention will be described in detail hereinunder. Examples and descriptions are illustrative of the invention and not restrictive and does not limit the scope of the invention.

<cylindrical body>

The cylindrical body of the present invention is a hollow cylindrical body which consists of a plurality of concentric layers and has an outer diameter of 0.5 to 50 mm and a thickness of 200 to 5,000 $\mu$m, and each layer is made of aliphatic polyester fibers having an average fiber diameter of 0.05 to 50 $\mu$m.

The cylindrical body of the present invention consists of a plurality of concentric layers. The thickness of each layer is preferably 30 to 500 $\mu$m, more preferably 50 to 250 $\mu$m. Each layer is preferably such that aliphatic polyester fibers are wound spirally with the axis of the cylindrical body as the center thereof.

The average fiber diameter of the aliphatic polyester fibers constituting the cylindrical body is 0.05 to 50 $\mu$m, preferably 0.2 to 20 $\mu$m, more preferably 0.2 to 10 $\mu$m. When the average fiber diameter is smaller than 0.05 $\mu$m, the strength of the cylindrical body cannot be maintained disadvantageously. When the average fiber diameter is larger than 50 $\mu$m, the specific surface area of each fiber becomes small and the number of cells adhering to the fiber becomes small disadvantageously. The average fiber diameter is the average of measurement values of fiber diameter at 20 sites obtained from an image taken by an optical microscope.

An example of the section of the cylindrical body of the present invention is shown in Fig. 6. Fig. 6 is a schematic diagram for explanation showing that irregularities are existent on the outer surface and inner surface of the cylindrical body of the present invention. The cylindrical body of the present invention has an outer diameter (11) of 0.5 to 50 mm, preferably 1 to 30 mm. The outer diameter is represented by a range from the smallest value to the largest value of the measurement values obtained by measuring 10 sites with a micrometer. The cylindrical body is hollow and the inner diameter (15) of the hollow portion is preferably 0.1 to 45 mm, more preferably 0.5 to 25 mm. The inner diameter is a difference between the measurement value of outer diameter and the measurement value of thickness.

The cylindrical body of the present invention has a thickness (14) of 200 to 5,000 $\mu$m, preferably 200 to 2,000 $\mu$m. When the thickness is smaller than 200 $\mu$m, the mechanical strength becomes low, whereby the cylindrical body is not preferred as a cell culture medium for a tissue having a high load such as a blood vessel. The thickness is represented by a range from the smallest value to the largest value of the measurement values obtained by cutting open the cylindrical body to prepare a sample having a length of 5 cm and a width of 1 cm and measuring 10 sites of the sample with a micrometer.

The tensile modulus of the cylindrical body is preferably in the range of 0.1 to 10 MPa. This is because the tensile modulus of the arterial vessel of the human body is actually 2 MPa (Clinical Engineering Library Series 2, p. 54 (Shuujunsha Co., Ltd.), physical properties of living bodies/mechanical engineering for medical purpose written by Kenji Ikeda and Hideteru Shimazu). When the tensile modulus is lower than 0.1 MPa, the cylindrical body may be broken because it cannot withstand the load of the blood. When the tensile modulus is higher than 10 MPa, compliance mismatch may occur at the time of implanting. The tensile modulus is obtained by cutting open the cylindrical body to prepare a sample having a length in the axial direction of 5 cm and a width in a circumferential direction of 1 cm and carrying out a tensile test on the sample in the axial direction.

The elastic recovery of the cylindrical body of the present invention is preferably 70 to 100 %. When the elastic recovery is lower than 70 %, the cylindrical body may be broken because it cannot withstand the load of the blood. Therefore, a cylindrical body having a tensile modulus of 0.1 to 10 MPa and an elastic recovery of 70 to 100 % is preferred. The elastic recovery is a value calculated from the following expression.

$$[L_0 - (L_{30} - L_0)]/L_0 \times 100 \ (\%)$$

$L_0$: length of cylindrical body (mm)
$L_{30}$: length of cylindrical body after it is pulled 30 times for 10 % displacement based on its length (mm)

The air permeability of the outermost layer of the cylindrical body is preferably 30 cm$^3$/cm$^2$·s or more, more preferably 70 to 250 cm$^3$/cm$^2$·s, much more preferably 70 to 200 cm$^3$/cm$^2$·s. When the air permeability of the outermost layer is 30 cm$^3$/cm$^2$·s or more, cell infiltration is satisfactory, which is preferred as a cell culture medium. The upper limit of air permeability of the outermost layer is substantially 250 cm$^3$/cm$^2$·s. The air permeability of the outermost layer is represented by the amount of permeated air measured in accordance with JIS-L1096 and JIS-R3420 under the condition that the differential pressure is 125 Pa and the thickness is 100 μm.

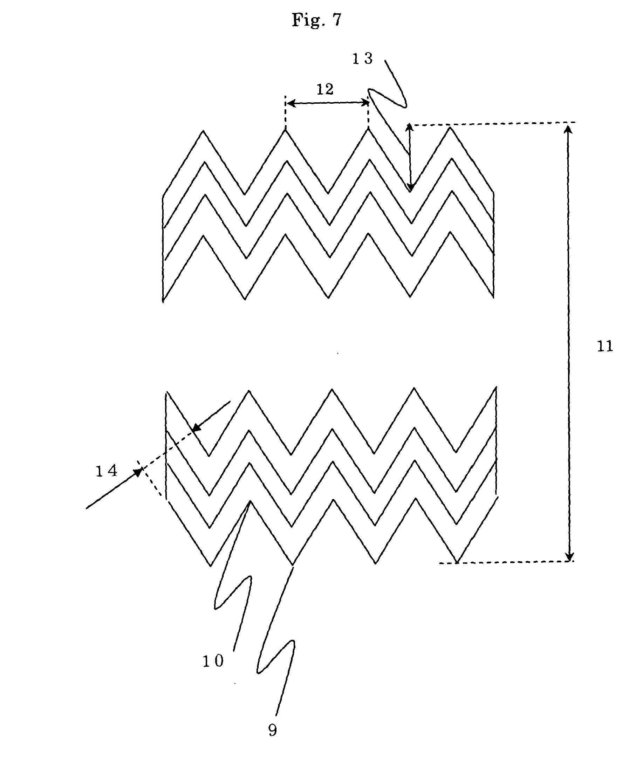

Preferably, the cylindrical body is a bellows-like cylindrical body having mountain portions (9) and valley portions (10) which are continuous in the axial direction, the interval between adjacent mountain portions (12) is 2 mm or less, and the valley portions have a depth (13) of 0.1 to 10 mm. The section of the bellows-like cylindrical body is shown in Fig. 7. When the interval between adjacent mountain portions (12) is larger than 2 mm, the elasticity of the cylindrical body becomes unsatisfactory. The interval between adjacent mountain portions and the interval between adjacent valley portions are obtained by measuring 10 sites with an optical microscope and represented by ranges from the largest value to the smallest value of the measurement values. Fig. 7 is a schematic diagram for explanation. The structure of the bellows-portion of the cylindrical body may be regular or irregular. The structure of the bellows-portion of the cylindrical body is irregular according to the manufacturing method, and the heights of the mountain portions, the depths of the valley portions and the intervals between them are not fixed.

In the present invention, at least one of the layers constituting the cylindrical body is preferably made different from the other layers. The actual vascular tissue has a three-layer structure consisting of an inner layer including endothelial cells covering the inner surface of the blood vessel, an intermediate layer composed of smooth muscle cells and an outer layer composed of fibroblasts all of which have different physical properties. As for the structure or physical properties of the tissue of each layer, the inner layer has a dense structure to control the selective permeation of a substance, the intermediate layer has an elastic function special to the blood vessel, and the outer layer has a sparse structure to take a nutritive substance from the outside of the blood vessel. Due to this constitution, the elastic function (elastic modulus and elastic recovery) and cell infiltration (high air permeability) special to the vascular tissue can be achieved. The expression "different from the other layers" means that the layer differs from the other layers in the type, molecular weight, copolymerization ratio and composition of a polymer constituting a fibrous structure as well as layer thickness and air permeability.

Examples of the aliphatic polyester include polylactic acid, polyglycolic acid, polycaprolactone, polydioxanone, trimethylene carbonate, polybutylene succinate, polyethylene succinate and copolymers thereof. Out of these, at least one selected from the group consisting of polylactic acid, polyglycolic acid, polycaprolactone and copolymers thereof is preferred.

At least one layer excluding the outermost layer of the cylindrical body used in the present invention is preferably made of aliphatic polyester fibers composed of a copolymer having a content of a recurring unit derived from caprolactone of 15 mol% or more to achieve the elastic function special to the vascular tissue. The copolymer is a copolymer of lactic acid and caprolactone. The copolymer is preferably composed of 50 to 85 mol% of a recurring unit derived from lactic acid and 15 to 50 mol% of a recurring unit derived from caprolactone.

The outermost layer of the cylindrical body preferably contains a recurring unit derived from glycolic acid to improve cell adhesion. The copolymer containing a recurring unit derived from glycolic acid is a copolymer of lactic acid and glycolic acid. The copolymer is preferably composed of 20 to 80 mol% of a recurring unit derived from lactic acid and 20 to 80 mol% of a recurring unit derived from glycolic acid. The outermost layer may contain a copolymer of lactic acid and caprolactone. The copolymer is preferably composed of 50 to 90 mol% of a recurring unit derived from lactic acid and 10 to 50 mol% of a recurring unit derived from caprolactone.

The outermost layer is preferably made of a composition comprising a copolymer of lactic acid and glycolic acid and a copolymer of lactic acid and caprolactone. The content of the former in the composition is preferably 50 to 90 wt% and the content of the latter in the composition is preferably 10 to 50 wt%. The former is preferably composed of 20 to 80 mol% of a recurring unit derived from lactic acid and 20 to 80 mol% of a recurring unit derived from glycolic acid. The latter is preferably composed of 50 to 90 mol% of a recurring unit derived from lactic acid and 10 to 50 mol% of a recurring unit derived from caprolactone.

The innermost layer of the cylindrical body preferably contains a recurring unit derived from glycolic acid to improve cell adhesion. The copolymer containing the recurring unit derived from glycolic acid is, for example, a copolymer of lactic acid and glycolic acid. The copolymer is preferably composed of 20 to 80 mol% of a recurring unit derived from lactic acid and 20 to 80 mol% of a recurring unit derived from glycolic acid. The innermost layer may contain a copolymer of lactic acid and caprolactone. The copolymer is preferably composed of 50 to 90 mol% of a recurring unit derived from lactic acid and 10 to 50 mol% of a recurring unit derived from caprolactone.

The innermost layer is preferably made of a composition comprising a copolymer of lactic acid and glycolic acid and a copolymer of lactic acid and caprolactone. The content of the former in the composition is preferably 50 to 90 wt% and the content of the latter in the composition is preferably 10 to 50 wt%. The former is preferably composed of 20 to 80 mol% of a recurring unit derived from lactic acid and 20 to 80 mol% of a recurring unit derived from glycolic acid. The latter is preferably composed of 50 to 90 mol% of a recurring unit derived from lactic acid and 10 to 50 mol% of a recurring unit derived from caprolactone.

Preferably, the cylindrical body consists of a first layer, a second layer and a third layer from the innermost side to the outermost side. In this cylindrical body, the first layer and the third layer are preferably made of a composition comprising 50 to 90 wt% of a copolymer of 20 to 80 mol% of lactic acid and 20 to 80 mol% of glycolic acid and 10 to 50 wt% of a copolymer of 50 to 90 mol% of lactic acid and 10 to 50 mol% of caprolactone. The second layer is preferably made of a copolymer of 50 to 90 mol% of lactic acid and 10 to 50 mol% of caprolactone.

The cylindrical body of the present invention may further contain a second component other than a bioabsorbable polymer. The component is preferably at least one selected from the group consisting of phosphatides, carbohydrates, glycolipids, steroids, polyamino acids, proteins and polyoxyalkylenes. Specific examples of the second component include phosphatides such as phosphatidyl choline, phosphatidyl ethanolamine, phosphatidyl serine and phosphatidyl glycerol, and/or carbohydrates such as polygalacturonic acid, heparin, chondroitin sulfate, hyaluronic acid, dermatan sulfate, chondroitin, dextran sulfate, sulfated cellulose, alginic acid, dextran, carboxymethylchitin, galactomannnan, Arabian gum, traganth gum, gellan gum, sulfated gellan, karaya gum, carageenan, agar, xanthane gum, curdlan, pullulan, cellulose, starch, carboxymethyl cellulose, methyl cellulose, glucomannan, chitin, chitosan, xyloglucan and lentinan, and/or glycolipids such as galactocerebroside, glucocerebroside, globoside, lactosylceramide, trihexosylceramide, paragloboside, galactosyldiacylglycerol, sulfoquinovosyldiacylglycerol, phophatidylinositol, glycosylpolyprenol phosphate and/or steroids such as cholesterol, cholic acid, sapogenin and digitoxin, and/or polyamino acids such as polyasparaginic acid, polyglutamic acid and polylysine, and/or proteins such as collagen, gelatin, fibronectin, fibrin, laminin, casein, keratin, sericin and thrombin, and/or polyoxyalkylenes such as polyoxyethylene alkyl ether, polyoxyethylene propylene alkyl ether and polyxoyethylene sorbitan ether, and/or cell growth factors such as FGF (fibroblast growth factor), EGF (epidermal growth factor) PDGF (plaque derived growth factor), TGF-β (β type genetic transformation growth factor), NGF (nerve growth factor), HGF (hepatic cell growth factor) and BMP (bone morphogenetic factor).

<manufacture of cylindrical body>

(process 1)

The cylindrical body of the present invention can be manufactured by the following steps:

(i) preparing dopes, each containing an aliphatic polyester and a volatile solvent, corresponding to the number of layers;
(ii) forming fibers from the dopes by an electrospinning process and winding them up onto a collector to obtain single-layer cylindrical bodies corresponding to the number of layers; and
(iii) laminating together the obtained cylindrical bodies.

(step (i))

The step (i) is to prepare dopes, each containing an aliphatic polyester and a volatile solvent, corresponding to the number of layers.

The aliphatic polyester has been described in the section for the cylindrical body. The volatile solvent is a substance which dissolves the aliphatic polyester, has a boiling point at normal pressure of 200°C or lower and is liquid at room temperature. Specific examples of the solvent include methylene chloride, chloroform, acetone, methanol, ethanol, propanol, isopropanol, toluene, tetrahydrofuran, 1,1,3,3-hexafluoroisopropanol, water, 1,4-dioxane, carbon tetrachloride, cyclohexane, cyclohexanone, N,N-dimethylformamide and acetonitrile. Out of these, methylene chloride, chloroform and acetone are particularly preferred from the viewpoints of the solubility of the aliphatic polyester. These solvents may be used alone or in combination of two or more. In the present invention, another solvent may be used in combination with the above solvents as long as the object of the present invention is not adversely affected.

The content of the aliphatic polyester in the dope is preferably 1 to 30 wt%, more preferably 2 to 20 wt%. When the content of the aliphatic polyester is lower than 1 wt%, it is difficult to form fibers disadvantageously due to the too low content. When the content is higher than 30 wt%, the obtained fibers become too large in size disadvantageously. The number of dopes corresponds to the number of layers. The type and content of the aliphatic polyester may be changed according to the dope.

(step (ii))

The step (ii) is to form fibers from the dopes by the electrospinning process and winding them up onto a collector to obtain single-layer cylindrical bodies corresponding to the number of layers. The plurality of cylindrical bodies manufactured independently form a plurality of concentric layers. Although the same type and amount of the aliphatic polyester may be used in the dopes, the dopes used in adjacent layers must be different from each other in the type and amount of the aliphatic polyester.

The electrospinning process is to manufacture a cylindrical body by ejecting a dope containing an aliphatic polyester and a volatile solvent into an electrostatic field formed between electrodes and spinning the dope toward the electrodes to wind a fibrous substance onto the collector. The expression "fibrous substance" means not only a fibrous substance from which the solvent of the solution has been distilled off but also a fibrous substance which still contains the solvent of the solution.

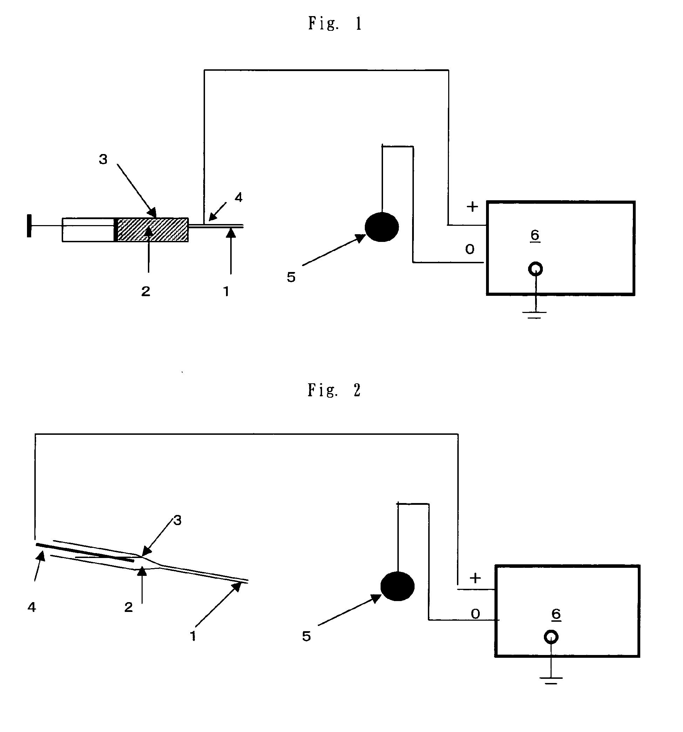

The electrospinning process can be carried out by using the apparatus shown in Fig. 1, for example. Fig. 1 shows an electrospinning apparatus comprising an ejection unit having a nozzle (1) equipped with an ejection-side electrode (4) and a storage tank (3), a collection-side electrode (5) and a high-voltage generator (6). A predetermined voltage is applied between the ejection-side electrode (4) and the collection-side electrode (5) by the high-voltage generator (6). In the apparatus shown in Fig. 1, the collection-side electrode (5) serves as a collector (7).

In the apparatus shown in Fig. 1, the dope (2) is filled into the storage tank (3), ejected into an electrostatic field through the nozzle (1) and spun by an electric field to form fibers which are collected on the collection-side electrode (5) to obtain a cylindrical body.

The electrodes consist of the ejection-side electrode (4) and the collection-side electrode (5). Any electrodes made of a metal, inorganic material or organic material may be used if they show conductivity. Electrodes having a conductive metal, inorganic or organic thin film on an insulating material may also be used. An electrostatic field is formed between a pair of electrodes or among a plurality of electrodes, and high voltage may be applied to any one of the electrodes. For example, two high-voltage electrodes which differ from each other in voltage value (for example, 15 kV and 10 kV) and an electrode connected to an earth may be used, or more than 3 electrodes may be used.

While the dope is spun toward the collection-side electrode (5), the solvent is evaporated according to conditions, thereby forming a fibrous substance. At normal room temperature, the solvent evaporates completely while the fibrous material is collected on the collection-side electrode (5). If the evaporation of the solvent is unsatisfactory, the dope may be spun under reduced pressure.

When a mandrel which is not planished is used as the collection-side electrode (5), the cylindrical body can be easily manufactured. When the cylindrical body is formed on the mandrel by the electrospinning process, the mandrel is preferably turned in a circumferential direction. By turning the collection-side electrode (5), a cylindrical body having uniform thickness can be formed. The revolution is preferably 1 to 1,000 rpm, more preferably 5 to 200 rpm.

The distance between the electrodes which depends on the amount of charge, the size of the nozzle, the ejection rate of the dope and the concentration of the dope is suitably 5 to 20 cm at about 10 kV. The potential of the applied static electricity is preferably 3 to 100 kV, more preferably 5 to 50 kV, much more preferably 5 to 30 kV.

When the dope is supplied into an electrostatic field from the nozzle, a plurality of nozzles may be used to increase the production rate of the fibrous substance. The inner diameter of the nozzle is preferably 0.1 to 5 mm, more preferably 0.1 to 2 mm.

The spinning temperature which depends on the evaporation behavior of the solvent and the viscosity of the liquid to be spun is generally 0 to 50°C.

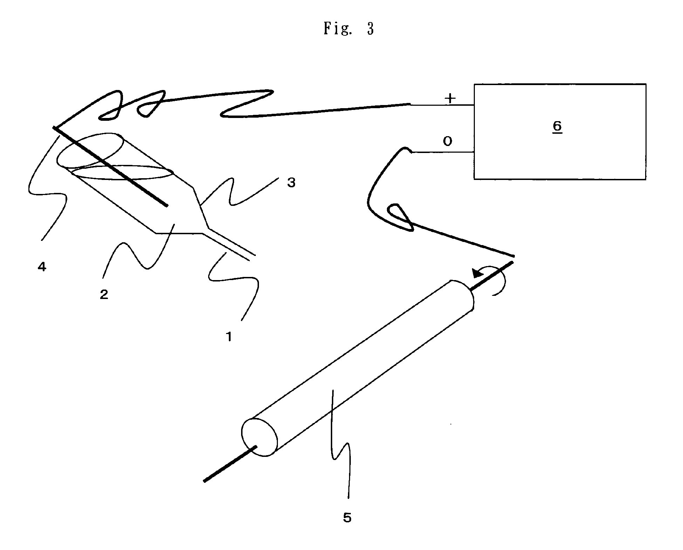

Fig. 2 shows an apparatus in which the ejection-side electrode (4) is inserted into the storage tank (3) having the nozzle (1) in place of the ejection unit. In this apparatus, the dope is scattered from the nozzle (1) toward the collection-side electrode (5) by adjusting the distance between the nozzle (1) and the collection-side electrode (5) in place of ejecting the dope with the ejection unit. Fig. 3 is a perspective view of Fig. 2.

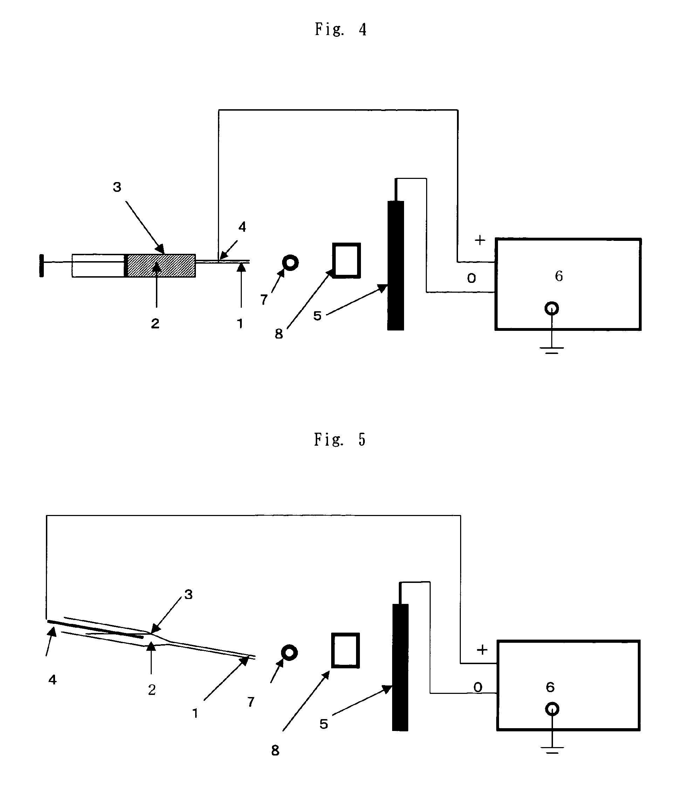

A collector (7) is installed between the ejection-side electrode (4) and the collection-side electrode (5) as shown in Fig. 4 to collect the fibrous substance. The collector (7) preferably has the same surface roughness as the mandrel used in the above collection-side electrode (5). When a belt-like collector (7) is installed between the electrodes, continuous production becomes possible. While the dope is spun toward the collector (7), the solvent evaporates according to conditions, and the fibrous substance is formed. At normal room temperature, while the fibrous substance is collected on the collector (7), the solvent evaporates completely. If the evaporation of the solvent is unsatisfactory, the dope may be spun under reduced pressure.

A cylindrical body having a plurality of layers at least one of which is different from the other layers can be obtained by changing the type and composition of the aliphatic polyester, the composition of the dope and electrostatic spinning conditions.

It is also preferred to use a static eraser (8) between the nozzle (1) and the collection-side electrode (5). By using the static eraser (8), the air permeability of the outermost layer can be increased.

(step (iii))

The step (iii) is to laminate together the obtained cylindrical bodies. The cylindrical body of the present invention can be manufactured by obtaining a plurality of cylindrical bodies and then laminating them together. The cylindrical body is plastic and can be manufactured by laminating together a plurality of cylindrical bodies of the same shape collected on the collector-side electrode (5) or the collector (7) of the same size. They can be easily laminated together by changing the outer diameter of the collection-side electrode (5) or the collector (7) to gradually increase the inner diameters of the outer layers. After the plurality of cylindrical bodies are laminated together, the layer is preferably heated. The heating temperature is preferably 40 to 90°C. When the fibers are collected on the collector continuously to form a thick fiber layer, the electrodes are insulated with the result of a reduction in spinning efficiency. However, when a plurality of single-layer cylindrical bodies are formed and laminated together as in the method of the present invention, the above problem does not occur.

(process 2)

The cylindrical body of the present invention can be manufactured by the following steps:

(i) preparing dopes, each containing an aliphatic polyester and a volatile solvent, corresponding to the number of layers;
(ii) forming fibers from a first dope by an electrospinning process and winding them up onto a collector to form a layer; and
(iii) forming a layer from the next dope on the obtained layer.

By repeating the above step (iii), a cylindrical body consisting of three or more layers can be obtained. The same number of cylindrical bodies as the number of layers are manufactured and laminated together in the process 1 whereas the same number of dopes as the number of layers are wound up onto the collector continuously to form a plurality of layers in the process 2. The process 1 and the process 2 are the same except for the above.

(bellows-like structure)

According to the present invention, a cylindrical body having a bellows-like structure can be obtained by extending the cylindrical body without impairing its elastic recovery. The extension rate is preferably 50 to 300 %. The expression "50 % extension" means that a length of 10 cm is extended to 15 cm. To obtain a cylindrical body having sufficiently high elastic recovery, the cylindrical body is preferably extended 50 % or more. When the cylindrical body is extended more than 300 %, the cylindrical body itself may be broken. When the cylindrical body is removed from the mandrel and the both ends of the cylindrical body are fixed to extend the cylindrical body, a cylindrical body having a bellows-like structure that the interval between mountain portions is 2 mm or less and valley portions have a depth of 0. 1 to 10 mm can be obtained. Fig. 7 is a sectional view of the cylindrical body having a bellows-like structure.
When the cylindrical body is to be removed from the collector (7) or the collection-side electrode (5), stress is applied only to one end of the cylindrical body, thereby making it possible to obtain the bellows-like structure. That is, one end of the cylindrical body is fixed, and the collector (7) or the collection-side electrode (5) is pulled out toward the direction of the fixed end to apply stress only to one end, thereby making it possible to obtain the bellows-like structure.

Examples

The following examples are provided for the purpose of further illustrating the present invention but are in no way to be taken as limiting.

(1) Polymers used in the examples are given below.

PLGA (50/50) : lactic acid (50 mol%)-glycolic acid (50 mol%) copolymer, intrinsic viscosity = 1.08 dL/g (in HFIP, 30°C), manufactured by Absorbable Polymers International Co., Ltd. PLCA (77/23): lactic acid (77 mol%)-caprolactone (23 mol%) copolymer, Mw = 2.5 x $10^5$, manufactured by Taki Chemical Co., Ltd.
PLCA (68/32): lactic acid (68 mol%)-polycaprolactone (32 mol%) copolymer, Mw = 1.75 x $10^5$, manufactured by Taki Chemical Co., Ltd.
Methylene chloride, ethanol: Wako Pure Chemical Industries, Ltd.

(2) The physical properties of the cylindrical body were measured by the following methods.

Average fiber diameter: 20 sites were measured by a digital microscope (VHX Digital Microscope of KEYENCE Co., Ltd.) to obtain the average of the measurement values as an average fiber diameter.

Outer diameter: 10 sites were measured by a micrometer (of Mitutoyo Corporation) to obtain a range from the smallest value to the largest value of the measurement values as an outer diameter.

Thickness: The cylindrical body was cut open to prepare a sample having a length of 5 cm and a width of 1 cm and 10 sites of the sample were measured by a micrometer to obtain a range from the smallest value to the largest value of the measurement values as a thickness.

Inner diameter: obtained from the difference between the measurement value of outer diameter and the measurement value of thickness.

Tensile elastic modulus: The cylindrical body was cut open to prepare a sample having a length in the axial direction of 5 cm and a width in the circumferential direction of 1 cm, and tensile force was applied to the cylindrical body in the axial direction to carry out a tensile test by Tensilon (EZ TEST: Shimadzu Corporation).

Elastic recovery: The cylindrical body was cut open to prepare a sample having a length in the axial direction of 5 cm and a width in the circumferential direction of 1 cm, and tensile force was applied to the cylindrical body in the axial direction to obtain $L_0$ and $L_{30}$ so as to calculate elastic recovery from the following equation.

$$[L_0 - (L_{30} - L_0)]/L_0 \times 100 \ (\%)$$

$L_0$: the length (mm) of the cylindrical body

$L_{30}$: the length (mm) of the cylindrical body after tensile force for 10 % elongation was applied to the cylindrical body 30 times in the axial direction

Air permeability of outermost layer: The cylindrical body was cut open to remove the outermost layer alone, and the outermost layer was cut into a 5 cm x 5 cm piece to measure its air permeability ($cm^3/cm^2 \cdot s$) by using the Fragile Permeameter (Toyo Seiki Co., Ltd.) in accordance with

JIS-L1096 and JIS-R3420 so as to calculate air permeability for a thickness of 100 $\mu$m.

Interval between mountain portions: The length denoted by 12 in Fig. 7 was measured at 10 sites by a digital microscope (VHX Digital Microscope of KEYENCE Co., Ltd.) to obtain a range.

Depth of valley portion: The length denoted by 13 in Fig. 7 was measured at 10 sites by a digital microscope (VHX Digital Microscope of KEYENCE Co., Ltd.) to obtain a range.

Reference Example 1 (preparation of dope)

(preparation of dope A)

1 g of PLCA (77/23) and 9 g of methylene chloride/ethanol (weight ratio of 8/1) were mixed together at room temperature (25°C) to prepare dope A having a concentration of 10 wt%.

(preparation of dope B)

1 g of PLCA (68/32) and 9 g of methylene chloride/ethanol (weight ratio of 8/1) were mixed together at room temperature (25°C) to prepare dope B having a concentration of 10 wt%.

(preparation of dope C)

0.8 g of PLGA (50/50), 0.2 g of PLCA (77/23) and 9 g of methylene chloride/ethanol (weight ratio of 8/1) were mixed together at room temperature (25°C) to prepare dope C having a concentration of 10 wt%.

Reference Example 2 (fabrication of cylindrical body A)

The apparatus shown in Fig. 2 was set up. The inner diameter of the nozzle (1) was 0.8 mm. The distance between the nozzle (1) and the collection-side electrode (5) was set to 10 cm. A stainless bar having an outer diameter of 4 mm and a length of 20 cm was used as the collection-side electrode (5).

The dope A was put into the storage tank (3), the voltages of the ejection-side electrode (4) and the collection-side electrode (5) were set to 14 kV, and the dope A was ejected toward the collection-side electrode (5) for 5 minutes while the collection-side electrode (5) was turned at 100 rpm to obtain a cylindrical body A. The same operation was repeated to obtain 5 cylindrical bodies A. The average fiber diameter of the fibers constituting the cylindrical body A was 4 $\mu$m, and the cylindrical body A had a length of 10 cm, an outer diameter of 4.1 to 4.2 mm, an inner diameter of 4 to 4.1 mm and a thickness of 90 to 110 $\mu$m. Reference Example 3 (fabrication of cylindrical body B)

The operation of Reference Example 2 was repeated except that the dope B was used in place of the dope A to fabricate a cylindrical body B. The average fiber diameter of the fibers constituting the cylindrical B was 4 $\mu$m, and the cylindrical body B had a length of 10 cm, an outer diameter of 4.1 to 4.2 mm, an inner diameter of 4 to 4.1 mm and a thickness of 90 to 110 $\mu$m.

Example 1 (A/A/A/A/A)

A stainless bar of the same diameter as the collection-side electrode 5 was placed within the first cylindrical body A. The second cylindrical body A was placed on the first cylindrical body A. Similarly, the second to fifth cylindrical bodies A were placed likewise. The cylindrical bodies A were soft and could be laminated together by pulling. Thereafter, the layer was heated at 70°C for 10 minutes to obtain a cylindrical body consisting of 5 layers cylindrical body A. The physical properties of the obtained cylindrical body are shown in Table 1.

Example 2 (A/A/B/A/A)

The operation of Example 1 was repeated except that the cylindrical body B was used in place of the third cylindrical body A from the bottom in Example 1 to obtain a cylindrical body consisting of the layers cylindrical body A/A/B/A/A. The characteristic properties of the cylindrical body having a plurality of layers obtained in Examples 1 and 2 are shown in Table 1.

Table 1

| | Outer diameter (mm) | Thickness ($\mu$m) | elastic modulus (MPa) | elastic recovery (%) | air permeability of outermost layer ($cm^3/cm^2 \cdot s$) |
|---|---|---|---|---|---|
| Ex.1 | 4.9 to 5.1 | 460 to 530 | 4.8 | 99.2 | 5 |
| Ex.2 | 4.9 to 5.1 | 470 to 550 | 2.5 | 99.0 | 5 |
| Arterial vessel*) | 25 | - | 2.0 | 100 | - |
| Ex.: Example \newline *) Clinical Engineering Library Series 2, p. 54 (Shuujunsha) refer to "Biological properties/mechanical engineering for medical application" written by Kenji Ikeda and Hideteru Shimazu | | | | | |

Example 3 (C/A/C)

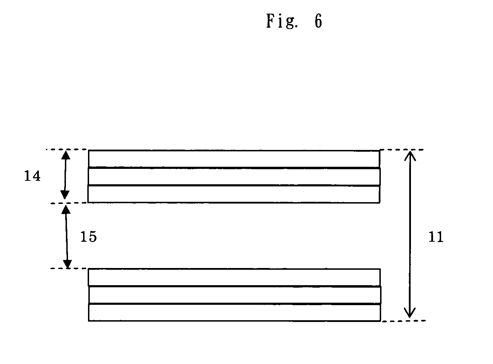

The apparatus shown in Fig. 5 was set up. The inner diameter of the nozzle (1) was 0.8 mm. The distance between the nozzle (1) and the collection-side electrode (5) was set to 10 cm. A stainless bar having an outer diameter of 4 mm and a length of 20 cm was used as the collection-side electrode (5). The collector (7) and the static eraser (8) were installed between the nozzle (1) and the collection-side electrode (5) as shown in Fig. 5.

(formation of first layer)

The dope C was put into the storage tank (3) and ejected toward the collection-side electrode (5) for 5 minutes to form a first layer. Voltage applied to the ejection-side electrode (4) and the collection-side electrode (5) was set to 14 kV. Fibers were collected while the collector (7) was turned at 100 rpm. The thickness of the first layer was 60 to 80 $\mu$m. The average fiber diameter of fibers constituting the first layer was 6 $\mu$m.

(formation of second layer)

Further, the dope A was put into the storage tank (3) in place of the dope C and ejected for 5 minutes to form a second layer. The thickness of the second layer was 60 to 80 $\mu$m. The average fiber diameter of fibers constituting the second layer was 4 $\mu$m.

(formation of third layer)

The dope C was put into the storage tank (3) in place of the dope A and ejected for 5 minutes to form a third layer so as to obtain a cylindrical body. The thickness of the third layer was 60 to 80 $\mu$m. The obtained cylindrical body had a length of 10 cm, an outer diameter of 4.4 to 4.5 mm and a thickness of 200 to 240 $\mu$m. The average fiber diameter of fibers constituting the third layer was 6 $\mu$m.

(formation of bellows-like structure)

The obtained cylindrical body was extended 100 % to obtain a cylindrical body having a bellows-like structure. The characteristic properties of the cylindrical body are shown in Table 2.

Example 4 (C/A/C)

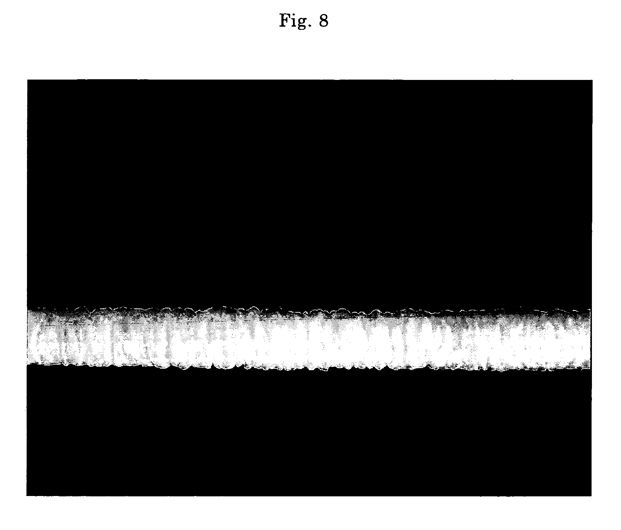

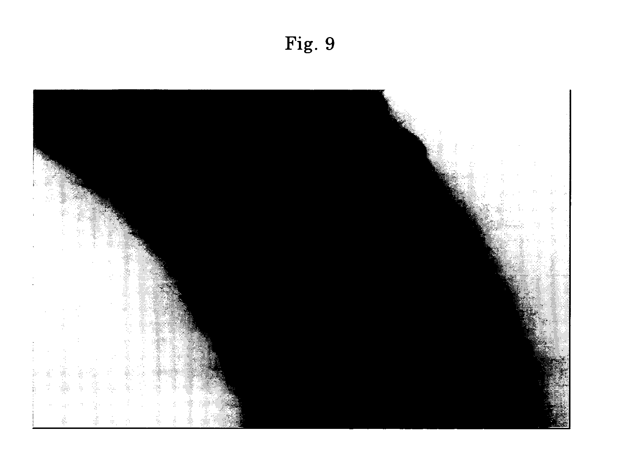

A cylindrical body was obtained in the same manner as in Example 3 except that the static eraser (8) was used to form the third layer. Fig. 8 shows a photo of the appearance of the obtained cylindrical body having a bellows-like structure. One division of a scale in Fig. 8 is 1 mm. The shapes of the mountain portions and the valley portions of the bellows are irregular. Fig. 9 shows a photo of the section of the cylindrical body. It is seen from the photo of Fig. 9 that the fiber density of the outermost layer is lower than those of the inner layers. The characteristic properties of the cylindrical body are shown in Table 2.

Example 5 (cylindrical body A/C)

(formation of first layer)

The apparatus shown in Fig. 5 was used to eject the dope A toward the collection-side electrode (5) for 10 minutes so as to form a first layer. The inner diameter of the nozzle (1) was set to 0.8 mm, the voltage was set to 12 kV, and the distance from the nozzle (1) to the collection-side electrode (5) was set to 20 cm. The thickness of the formed first layer was 180 to 220 $\mu$m. The average fiber diameter of fibers constituting the first layer was 4 $\mu$m.

(formation of second layer)

Further, the dope C was ejected toward the collection-side electrode (5) for 1 minute to form a second layer so as to obtain a cylindrical body. The thickness of the second layer was 40 to 80 $\mu$m. The obtained cylindrical body had a length of 10 cm, an outer diameter of 4.4 to 4.5 mm and a thickness of 220 to 260 $\mu$m. The average fiber diameter of fibers constituting the second layer was 6 $\mu$m.

(formation of bellows-like structure)

One end of the cylindrical body was fixed by holding it with a finger, and the collection-side electrode (5) was held with a finger and pulled toward the fixed side to obtain a cylindrical body having a bellows-like structure. The characteristic properties of the obtained cylindrical body are shown in Table 2.

Table 2

| | Outer diameter (mm) | Thickness ($\mu$m) | elastic modulus (MPa) | elastic recovery (%) | interval between mountain portions (mm) | depth of valley portion (mm) | air permeability of outermost layer ($cm^3/cm^2 \cdot s$) |
|---|---|---|---|---|---|---|---|
| Ex.3 | 4.4 to 4.5 | 200 to 240 | 0.35 | 88 | 0.5 to 2 | 0.5 to 1 | 84 |
| Ex.4 | 4.4 to 4.5 | 220 to 260 | 0.21 | 85 | 0.5 to 2 | 0.5 to 1 | 149 |
| Ex.5 | 5.4 to 5.5 | 200 to 240 | 0.33 | 80 | 0.5 to 2 | 0.5 to 1 | 98 |
| Ex.: Example | | | | | | | |

Example 6

Mouse embryo fibroblasts (NIH3T3 cells) (manufactured by ATCC) were planted in the cylindrical body fabricated in Example 4 at a density of 1 x $10^6$ per $cm^2$ and cultured under 5 % $CO_2$ and 37°C environment for 7 days by using a culture fluid (DMEM of Gibco Co., Ltd.) containing 10 % of bovine fetal serum (HyClone Co., Ltd.). When the cylindrical body was cut open to carry out MTT assay (Funakoshi Co., Ltd.), the growth of cells could be confirmed.

Industrial Feasibility

The cylindrical body of the present invention can be used as an artificial blood vessel as well as a cell culture medium because it shows physical properties similar to those of a vascular tissue.

**Claims**

1.  A hollow cylindrical body which consists of a plurality of concentric layers and has an outer diameter of 0.5 to 50 mm, a thickness of 200 to 5,000 $\mu$m, wherein
    each of the layers is made of aliphatic polyester fibers having an average fiber diameter of 0.05 to 50 $\mu$m.

2.  The cylindrical body according to claim 1, wherein each of the layers is manufactured by winding the aliphatic polyester fibers spirally with the axis of the cylindrical body as the center thereof.

3.  The cylindrical body according to claim 1 which has a tensile elastic modulus of 0.1 to 10 MPa and an elastic recovery of 70 to 100 %.

4.  The cylindrical body according to claim 1, wherein the air permeability of the outermost layer is 30 $cm^3/cm^2 \cdot s$ or more when the outermost layer has a thickness of 100 $\mu$m at a differential pressure of 125 Pa.

5.  The cylindrical body according to claim 1, wherein at least one layer is made of aliphatic polyester fibers different from those of the other layers.

6.  The cylindrical body according to claim 5, wherein the aliphatic polyester is at least one selected from the group consisting of polylactic acid, polyglycolic acid, polycaprolactone and copolymers thereof.

7.  The cylindrical body according to claim 1, wherein at least one layer excluding the outermost layer is made of aliphatic polyester fibers composed of a copolymer having a content of a recurring unit derived from caprolactone of 15 mol% or more.

8.  The cylindrical body according to claim 1 which is a bellows-like cylindrical body having mountain portions and valley portions continuous in an axial direction, the interval between the mountain portions being 2 mm or less and the valley portions having a depth of 0.1 to 10 mm.

9.  A method of manufacturing a hollow cylindrical body consisting of a plurality of concentric layers, comprising the steps of:

    (i) preparing dopes, each containing an aliphatic polyester and a volatile solvent, corresponding to the number of layers;
    (ii) forming fibers from the dopes by an electrostatic spinning process and winding them up onto a collector to obtain single-layer cylindrical bodies corresponding to the number of layers; and
    (iii) laminating together the obtained cylindrical bodies.

10. The method of manufacturing a cylindrical body according to claim 9, wherein the plurality of cylindrical bodies are laminated together and heated.

11. The method of manufacturing a cylindrical body according to claim 9, comprising the step of extending the obtained cylindrical body.

12. A method of manufacturing a hollow cylindrical body consisting of a plurality of concentric layers, comprising the steps of:

    (i) preparing dopes, each containing an aliphatic polyester and a volatile solvent, corresponding to the number of layers;
    (ii) forming layers from a first dope by an electrospinning process and winding them up onto a collector to form a layer; and

(iii) forming a layer from the next dope on the obtained layer.

13. The method of manufacturing a cylindrical body according to claim 12, wherein the step (iii) is repeated.

14. The method of manufacturing a cylindrical body according to claim 12 comprising the step of extending the obtained cylindrical body.

Fig. 1

Fig. 2

Fig. 3

Fig. 4

Fig. 5

Fig. 6

Fig. 7

Fig. 8

Fig. 9

## INTERNATIONAL SEARCH REPORT

| | International application No. |
|---|---|
| | PCT/JP2005/021638 |

| A. CLASSIFICATION OF SUBJECT MATTER |
|---|
| *A61F2/06*(2006.01), *A61L27/00*(2006.01) |

According to International Patent Classification (IPC) or to both national classification and IPC

| B. FIELDS SEARCHED |
|---|

Minimum documentation searched (classification system followed by classification symbols)
A61F2/06, A61L27/00

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| Y | JP 2004-321484 A  (Kyushu TLO Co., Ltd.),<br>18 November, 2004 (18.11.04),<br>Par. Nos. [0010] to [0013], [0035], [0047];<br>Figs. 4 to 5<br>(Family: none) | 1-14 |
| Y | JP 59-181149 A  (Intermedicat GmbH.),<br>15 October, 1984 (15.10.84),<br>Page 6, lower right column, line 1 to page 7,<br>lower right column, line 15; Figs. 1 to 4<br>& US 4474630 A          & GB 2015118 A<br>& DE 2806030 A          & FR 2416686 A | 1-14 |
| Y | JP 1-242061 A  (Kobita, Corp.),<br>27 September, 1989 (27.09.89),<br>Full text; all drawings<br>(Family: none) | 2 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered  to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 January, 2006 (11.01.06) | 24 January, 2006 (24.01.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2005/021638

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 1-155860 A  (Ube Industries, Ltd.),<br>19 June, 1989 (19.06.89),<br>Page 3, upper right column, line 10 to lower<br>left column, line 20<br>(Family: none) | 8,10,11,14 |
| A | JP 2004-290133 A  (Teijin Ltd.),<br>21 October, 2004 (21.10.04),<br>Full text; all drawings<br>(Family: none) | 1-14 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 52110977 A

- JP 2004321484 A

**Non-patent literature cited in the description**

- *Biomaterials,* 2004, vol. 25, 877